# EUROPEAN PATENT SPECIFICATION

(11) **EP 2 404 541 B1**
(45) Date of publication and mention of the grant of the patent: **17.06.2015**
(21) Application number: 10748744.9
(22) Date of filing: 02.03.2010
(51) Int. Cl.: A61B 1/00, A61B 1/04

(54) **ENDOSCOPE with cable winding element**
ENDOSKOP mit Kabelwicklungvorrichtung
ENDOSCOPE avec dispositif d'enroulement de cable

(30) Priority: 02.03.2009 JP 2009048148
(43) Date of publication of application: 11.01.2012
(73) Proprietor: OLYMPUS MEDICAL SYSTEMS CORP., Tokyo 151-0072 (JP)
(72) Inventor: KISHIOKA, Shigeyasu, Tokyo 192-8512 (JP)
(74) Representative: Gunzelmann, Rainer
(86) International application number: PCT/JP2010/053345
(87) International publication number: WO 2010/101149

(56) References cited:
- JP-A- 9 243 934
- JP-A- 2001 305 435
- JP-A- 2008 043 726
- US-A- 4 759 346
- US-A1- 2003 050 534
- US-A1- 2007 225 556

## Description

### Technical Field

The present invention relates to an endoscope wherein an optical image conducting member is inserted through an endoscope main portion and is configured to conduct an optical image, a display portion is coupled to an operation portion of the endoscope main portion and is configured to display the imaged optical image, and the display portion is configured to be moved relative to the operation portion.

### Background Art

In Jpn. Pat. Appln. KOKAI Publication No. 2006-43094 discloses an endoscope apparatus. In the endoscope apparatus, an operation portion configured to be grasped and operated by an operator is coupled to the proximal end portion of an elongated insertion portion configured to be inserted into the body. An objective lens is provided in the distal end portion of the insertion portion, the distal end portion of an image guide is connected to the objective lens, and the image guide is inserted through the insertion portion and put into the operation portion. An observation image is formed by the objective lens, and the formed optical image is conducted by the image guide. An image display apparatus is coupled to the proximal end portion of the operation portion and is configured to display the optical image conducted by the image guide and imaged. The image display apparatus is configured to be rotated and titled relative to the operation portion such that it is easy for an observer to observe the optical image.

Document US 4,759,346 discloses an endoscope device provided with provided with two kinds of electronscopes different at least in the length of the insertion part and each housing a solid state imaging device on the tip side of the insertion part and a camera controlling unit making the respective electronscopes fittable and housing a means of impressing driving pulses for reading out signals on said solid state imagine device and a video signal processing means taking in and processing the output signal read out. The length of the signal cable electrically connecting the above mentioned solid state imaging device and camera controlling unit in each electronscope is made equal to that in the other electronscope.

Another prior art endoscope is known from JP 2008 043 726.

### Disclosure of Invention

In the endoscope apparatus disclosed in Jpn. Pat. Appln. KOKAI Publication No. 2006-43094, in the case where an electric cable extends from an inner space of the operation portion into an inner space of the image display apparatus and the connecting end portion of the electric cable is connected to the image display apparatus, it is necessary to form a play formed by additional length of the electric cable so as not to prevent rotation or the like of the image display apparatus relative to the operation portion. When rotation and so on of the image display apparatus relative to the operation portion is performed, the part forming the play in the electric cable is acted following the image display apparatus, and the part forming the play of the electric cable may be brought into contact with and damage the image guide.

The present invention has been made in view of the above-mentioned problem, and an object of the present invention is to provide an endoscope wherein an optical image conducting member is prevented from being damaged.

An endoscope according to the invention is defined in claim 1.

In the endoscope according to the invention, the restricting mechanism restricts movement of the part forming the play in the electric connecting member toward the optical image conducting member. Therefore, contact of the part forming the play in the electric connecting member with the optical image conducting member is avoided, and the optical image conducting member is prevented from being damaged.

Further disclosed is an endoscope which is characterized in that the restricting mechanism includes: a wound portion provided in the inner space of the operation portion or the inner space of the display portion; and a winding portion being a part of the electric connecting member and wound around the wound portion to form the play.

In this endoscope, the part forming the play in the electric connecting member is wound around the wound portion to form the winding portion, and thus, movement of the part forming the play toward the optical image conducting member is restricted. Moreover, when the display portion is moved relative to the operation portion and the winding portion is acted following the display portion, the winding portion is acted regularly, that is, is wound around or is released from the wound portion, the part forming the play is prevented from being acted irregularly to be brought into contact with the optical image conducting member.

Still further, an endoscope is characterized in that the restricting mechanism further includes a winding portion receiving portion provided in the inner space of the operation portion or the inner space of the display portion, arranged between the winding portion and the optical image conducting member and configured to support the winding portion.

In this endoscope, even when the winding portion forming the play is moved toward the optical image conducting member, the winding portion receiving portion supports the winding portion, and movement of the winding portion toward the optical image conducting member beyond the winding portion receiving portion is restricted. Moreover, even when the winding portion is acted irregularly toward the optical image conducting member while the display portion is moved relative to the operation portion and the winding portion is acted following the display portion, the winding portion receiving portion supports the winding portion, and action of the winding portion toward the optical image conducting member beyond the winding portion receiving portion is restricted Therefore, contact of the part forming the play in the electric connecting member with the optical image conducting member is certainly avoided, and the optical image conducting member is sufficiently prevented from being damaged.

Still further, an endoscope is characterized in that the restricting mechanism includes: a restricting portion provided at the electric connecting member; a restricting receiving portion provided in the inner space of the operation portion or the inner space of the display portion and configured to support the restricting portion to restrict movement of the restricting portion toward the optical image conducting member, and the electric connecting member includes a play portion formed by a part between the restricting portion and the connecting end portion and forming the play.

In this endoscope, the restricting portion of the electric connecting member is brought into contact with the restricting receiving portion, and movement of the restricting portion toward the optical image conducting member is restricted, and thus, movement of the play portion between the restricting portion and the connecting end portion toward the optical image conducting member is restricted.

Still further, an endoscope is **characterized in that** the restricting mechanism includes a play portion receiving portion provided in the inner space of the display portion or the inner space of the operation portion, arranged between the play portion and the optical image conducting member and configured to support the play portion.

In this endoscope, even when the play portion forming the play is moved toward the optical image conducting member, the play portion receiving portion supports the play portion, and movement of the play portion toward the optical image conducting member beyond the play portion receiving portion is restricted. Moreover, even when the play portion is acted irregularly toward the optical image conducting member when the display portion is moved relative to the operation portion and the play portion is acted following the display portion, the play portion receiving portion supports the play portion, and action of the play portion toward the optical image conducting member beyond the play portion receiving portion is restricted. Therefore, contact of the part forming the play in the electric connecting member with the optical image conducting member is certainly avoided, and the optical image conducting member is sufficiently prevented from being damaged.

### Brief Description of Drawings

FIG. 1 is a perspective view showing an endoscope according to a first embodiment of the present invention;
FIG. 2 is a longitudinal cross-sectional view showing an operation portion and a monitor portion according to the first embodiment of the present invention;
FIG. 3 is a view showing the monitor portion according to the first embodiment of the present invention when viewing in a direction of the arrow III in FIG. 2;
FIG. 4 is a transverse cross-sectional view showing the monitor portion according to the first embodiment of the present invention along the IV-IV line in FIG. 2;
FIG. 5 is a longitudinal cross-sectional view showing the monitor portion according to the first embodiment of the present invention along the V-V line in FIG. 4;
FIG. 6 is a longitudinal cross-sectional view showing an operation portion and a monitor portion according to a second embodiment of the present invention;
FIG. 7 is a longitudinal cross-sectional view showing an operation portion and a monitor portion according to a third embodiment of the present invention;
FIG. 8 is a perspective view showing a valve unit according to a first referential embodiment of the present invention;
FIG. 9 is a partially longitudinal cross-sectional side view showing the valve unit according to the first referential embodiment of the present invention along the IX-IX line in FIG. 11A;
FIG. 10A is a perspective view showing the valve unit according to the first referential embodiment of the present invention in an attachment state;
FIG. 10B is a perspective view showing the valve unit according to the first referential embodiment of the present invention in a detachment state;
FIG. 11A is a partially transverse cross-sectional top view showing the valve unit according to the first referential embodiment of the present invention in the attachment state along the XI-XI line in FIG. 9;
FIG. 11B is a partially transverse cross-sectional top view showing the valve unit according to the first referential embodiment of the present invention in the detachment state along the XI-XI line in FIG. 9;
FIG. 12A is a partially transverse cross-sectional top view showing the valve unit according to the first referential embodiment of the present invention in the attachment state along the XII-XII line in FIG. 9;
FIG. 12B is a partially transverse cross-sectional top view showing the valve unit according to the first referential embodiment of the present invention in the detachment state along the XII-XII line in FIG. 9;
FIG. 13 is a top view showing a distal end rigid portion according to a second referential embodiment of the present invention;
FIG. 14 is a longitudinal cross-sectional view showing a distal unit according to the second referential embodiment of the present invention along the XIV-XIV line in FIG. 13;
FIG. 15 is a transverse cross-sectional view showing the distal unit according to the second referential embodiment of the present invention along the XV-XV line in FIG. 14;
FIG. 16 is a top view showing a nozzle holding unit according to the second referential embodiment of the present invention;
FIG. 17 is a front view showing the nozzle holding unit according to the second referential embodiment of the present invention; and
FIG. 18 is a top view showing a distal cover member according to the second referential embodiment of the present invention.

### Best Mode for Carrying Out the Invention

Each embodiment of the present invention will be explained referring to the drawings.

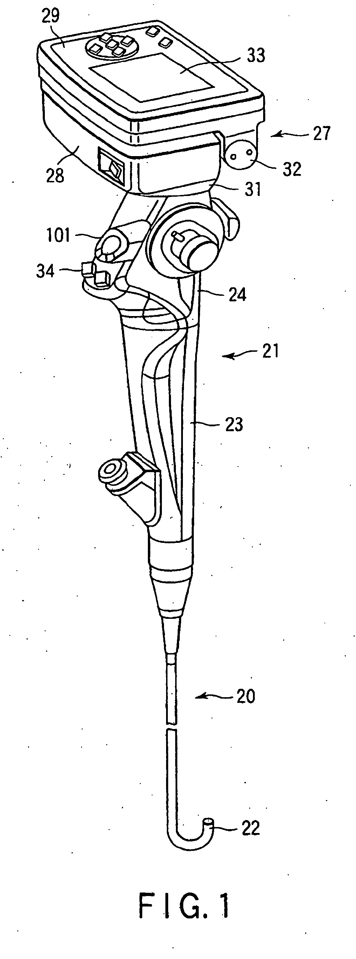

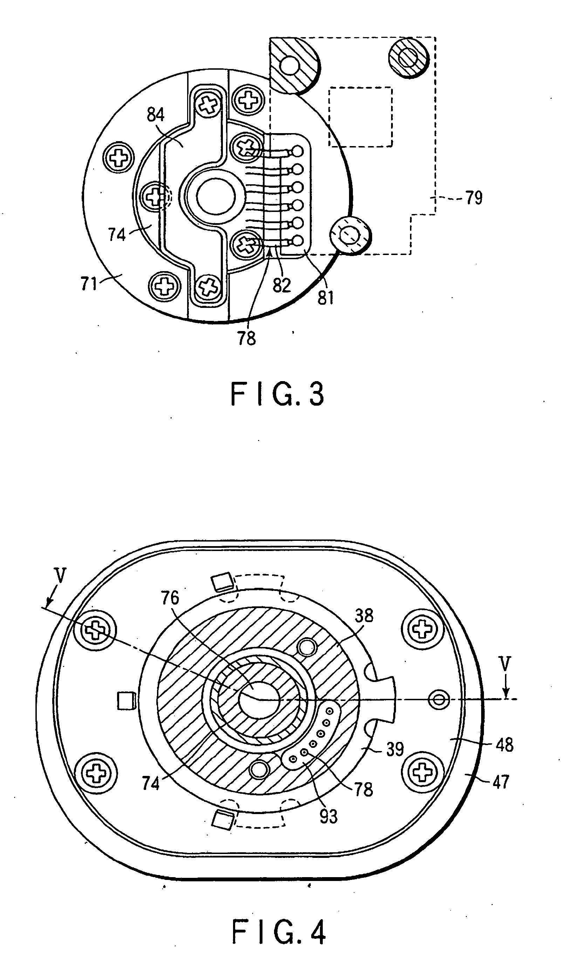

Referring to FIGS. 1 to 5, a first embodiment of the present invention will be explained.

Referring to FIG. 1, an outline structure of a carrying type of endoscope with a monitor will be explained.

The endoscope includes an elongated insertion portion 20 configured to be inserted into the body. A distal end rigid portion 22 is formed at the distal end portion of the insertion portion 20, and an operation portion 21 is coupled to the proximal end portion of the insertion portion 20. A grasping portion 23 is formed at the distal end side part of the operation portion 21 and is configured to be grasped by an operator, and an operation portion main portion 24 is formed at the proximal end side part of the operation portion 21 and is configured to be operated by the operator. An illumination unit is provided within the grasping portion 23, and a light guide fiber extends from the illumination unit, is inserted through the operation portion 21 and the insertion portion 20 and reaches an illumination optical system in the distal end rigid portion 22. An illumination light is produced by the illumination unit, conducted through the light guide fiber, and emitted from the illumination optical system to an observation target. Moreover, an objective optical system is provided within the distal end rigid portion 22, the distal end portion of an image guide fiber 26 as an optical image conducting member is coupled to the objective optical system, and the image guide fiber 26 is inserted through the insertion portion 20 and put into the operation portion 21. An observation image is formed on the distal end surface of the image guide fiber 26 by the objective optical system, and the formed optical image is conducted through the image guide fiber 26 to the proximal end surface of the image guide fiber 26.

A monitor portion 27 as a display portion is coupled to the proximal end portion of the operation portion 21. In the monitor portion 27, a rectangular thick plate-shaped imaging assembly 28 and a rectangular thick plate-shaped monitor assembly 29 are arranged perpendicular to the axial direction and overlapped with each other from the distal end side to the proximal end side. The operation portion 21 and the imaging assembly 28 are coupled with each other by a rotational coupling mechanism 31, and the imaging assembly 28, that is, the whole monitor portion 27 is configured to be rotated about the central axis of the endoscope as a rotational axis relative to the operation portion 21. The imaging assembly 28 and the monitor assembly 29 are coupled with each other by a tilting coupling mechanism 32, and the monitor assembly 29 is configured to be tilted relative to the imaging assembly 28 so as to rise up from the imaging assembly 28. A monitor 33 is provided at the proximal end surface portion of the monitor assembly 29 and configured to display the optical image conducted by the image guide fiber 26 and imaged as an observation image. It is possible to arrange the monitor 33 at a position such that an operator and the like can easily observe the monitor 33, by rotating the monitor portion 27 relative to the operation portion 21 and tilting the monitor assembly 29 relative to the imaging assembly 28.

Moreover, operation switches 34 are provided at the operation portion main portion 24 of the operation portion 21 and are configured to operate the monitor portion 27. A release switch configured to release the observation image, a frieze switch configured to frieze the observation image and so forth are used as the operation switchs 34.

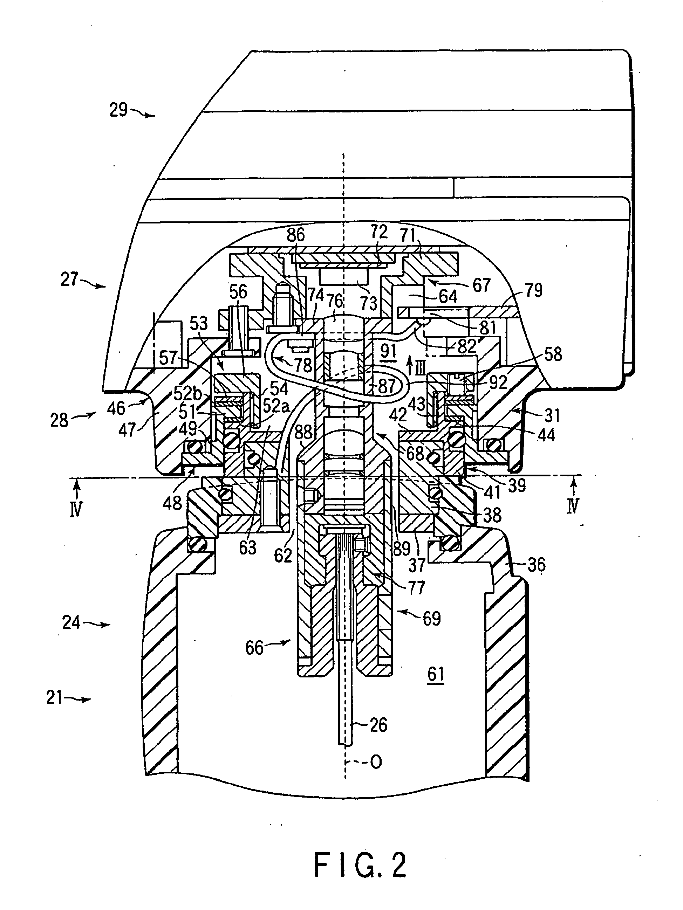

Referring to FIGS. 2 to 5, the operation portion 21 and the monitor portion 27 will be explained in details.

Referring to FIG. 2, the rotational coupling mechanism 31 will be explained in details, which couples rotatably the operation portion 21 and the imaging assembly 28 with each other. A circular ring-shaped proximal end wall 37 is formed at an operation housing 36 forming the operation portion 21 and is orthogonal to the central axis of the endoscope. A thick circular cylinder-shaped support member 38 is coaxially coupled on the proximal end side of the proximal end wall 37. An operation coupling member 39 is coaxially provided on the outside of the proximal end side part of the support member 38. The operation coupling member 39 includes a circular cylinder-shaped sliding receiving portion 41. A thin circular ring-shaped covering portion 42 is formed at the proximal end portion of the inner peripheral surface of the sliding receiving portion 41, protrudes inwardly in the radial direction and extends over the whole periphery. A thin circular cylinder-shaped holding receiving portion 43 is formed at the radially inner end part of the proximal end surface of the sliding receiving portion 41 and protrudes toward the proximal end side. A putting receiving surface 44 is formed by the radially outside part of the proximal end surface of the sliding receiving portion 41. The sliding receiving portion 41 is fitted on the outside of and fixed to the proximal end side part of the outer peripheral surface of the support member 38, and the covering portion 42 covers and is fixed to the proximal end surface of the support member 38. On the other hand, a thick circular cylinder-shaped supporting portion 47 is formed at the distal end side part of an imaging housing 46 forming the imaging assembly 28 of the monitor portion 27. An imaging coupling member 48 is coaxially provided at the inside of the distal end side part of the supporting portion 47. The imaging coupling member 48 include a circular cylinder-shaped sliding portion 49, and a circular ring-shaped putting portion 51 is formed at the proximal end portion of the inner peripheral surface of the sliding portion 49, protrudes inwardly in the radial direction and extends over the whole periphery. The sliding portion 49 of the imaging coupling member 48 is fitted on the inside of and fixed to the distal end side part of the inner peripheral surface of the supporting portion 47, and the sliding portion 49 is provided on the outside of the sliding receiving portion 41 of the operation coupling member 39 and is configured to be rotated about the central axis of both of the coupling members 39 and 48 as a rotational axis O relative to the sliding receiving portion 41. The putting portion 51 of the imaging coupling member 48 is put at the putting receiving surface 44 of the operation coupling member 39 via a first sliding washer 52a. A circular ring-shaped sandwiching member 57 is put at the proximal end surface of the putting portion 51 via a second sliding washer 52b. The first sliding washer 52a, the putting portion 51, the second sliding washer 52b and the sandwiching member 57 are provided at the outside of the holding receiving portion 43 of the operation coupling member 39. A pushing member 53 is provided at the proximal end side part of the holding receiving portion 43. The pushing member 53 includes a thin circular cylinder-shaped holding portion 54, and a circular ring-shaped pushing portion 56 is formed at the proximal end portion of the outer peripheral surface of the holding portion 54, protrudes outwardly in the radial direction and extends over the whole periphery. The holding portion 54 of the pushing member 53 is fitted on the inside of and fixed to the holding receiving portion 43. The pushing portion 56 of the pushing member 53 overhangs beyond the holding receiving portion 43 and faces the sandwiching member 57. The threaded bore passes through the pushing portion 56 in the axial direction, and a fixing screw 58 is screwed into the threaded bore. The fixing screw 58 is screwed toward the distal end and pushes the sandwiching member 57 toward the distal end, and thus, the putting portion 51 of the imaging coupling member 48 is sandwiched and held in the axial direction between the sandwiching member 57 and the putting receiving surface 44 of the operation coupling member 39. The sandwiching force is appropriately adjusted by means of the fixing screw 58 such that the imaging coupling member 48 can not be moved in the axial direction relative to the operation coupling member 39 and the imaging coupling member 48 is rotatable relative to the operation coupling member 39 by applying rotational operation force larger than a certain force.

Referring to FIG. 2, inner spaces of the operation portion 21 and the imaging assembly 28 will be explained.

An operation space 61 is formed by the inner space of the operation housing 36. Moreover, a distal end side coupling space 62 is formed by each of the central bores of the proximal end wall 37 of the operation housing 36, the support member 38, and the covering portion 42 of the operation coupling member 39. Furthermore, a proximal end side coupling space 63 is defined by the covering portion 42, the pushing member 53 and the proximal end side part of the supporting portion 47. The minimum outside diameter of the proximal end side coupling space 63 is mainly defined by the inside diameter of the pushing member 53 and is larger than the outside diameter of the distal end side coupling space 62 approximately by the radial width of the covering portion 42 of the operation coupling member 39. The proximal end side part of the imaging housing 46 is in the shape of a rectangular parallelepiped box and forms an imaging space 64.

Referring to FIG. 2, an imaging unit 66 will be explained, which is configured to image an optical image.

An imaging unit 66 protrudes from the inner surface of the proximal end wall of the imaging housing 46 and extends along the rotational axis O from the proximal end side to the distal end side. The imaging unit 66 is coaxial with the rotational axis O, and each component of the imaging unit 66 mentioned below is arranged coaxially with the rotational axis O. In the imaging unit 66, an imaging element portion 67, a relay lens portion 68 and a fiber fixing portion 69 are provided in order from the proximal end side to the distal end side. The imaging element portion 67, the relay lens portion 68 and the fiber fixing portion 69 are arranged in the imaging space 64, the coupling spaces 62 and 63 and the operation space 61, respectively. In the imaging element portion 67, a circular cylinder-shaped mounting frame 71 protrudes from the inner surface of the proximal end wall of the imaging housing 46. An imaging element 72 covers the inner surface of the proximal end wall within the mounting frame 71, and a cover glass 73 covers the imaging element 72. In the relay lens portion 68, the proximal end surface of the proximal end outward flange of the lens frame 74 covers and is fixed to the distal end surface of the mounting frame 71. Relay lenses 76 are arranged in order in the axial direction within the lens frame 74. In the fiber fixing portion 69, the proximal end portion of a circular cylinder-shaped fixing frame portion 77 is coupled to the distal end portion of the relay lens portion 68. The coupling end portion of the proximal end portion of the image guide fiber 26 is fitted into the central bore of the fixing frame portion 77 from the distal end side and fixed to the fixing frame portion 77 by means of a fixing screw and others, and the proximal end surface of the image guide fiber 26 faces the distal end surface of the most distal relay lens 76. The proximal end side part of the image guide fiber 26 is arranged coaxially with the rotational axis O. An optical image conducted through the image guide fiber 26 enters from the proximal end surface of the image guide fiber 26 through the relay lenses 76 into the imaging element 72, imaged by the imaging element 72 to be converted to an imaging signal, and is displayed on the monitor 33 of the monitor portion 27 as an observation image.

Referring to FIGS. 2 to 5, an electrical cable 78 as an electric connecting member will be explained.

A circuit board 79 is provided in the imaging space 64 of the imaging housing 46, adjacent to the mounting frame 71 of the imaging unit 66, orthogonal to the axial direction of the endoscope and configured to control the switches 34 or the like. Electric contact portions 81 are formed on the side close to the mounting frame 71 in the distal end surface of the circuit board 79. Connecting end portions 82 of the proximal end portions of the electrical cables 78 are coupled to the electric contact portions 81 by means of soldering and so on.

A proximal end outward flange of the lens frame 74 is concentrically arranged on the radial inside of the distal end outward flange of the mounting frame 71. A plate-shaped protecting member 83 made of rubber and a clamp member 84 are overlapped with each other and put at the position opposite to the electric contact portions 81 relative to the rotational axis O at the distal end surfaces of the distal end outward flange of the mounting frame 71 and the proximal end outward flange of the lens frame 74. Both end portions of the protecting member 83 and the clamp member 84 are fixed to the distal end surface of the distal end outward flange of the mounting frame 71, and the protecting member 83 and the clamp member 84 extend across the distal end surface of the proximal end outward flange of the lens frame 74. The distal end surface of the proximal end outward flange of the lens frame 74 is arranged closer to the proximal end side than the distal end surface of the distal end outward flange of the mounting frame 71, the electrical cables 78 extended from the electric contact portions 81 are sandwiched between and held by the protecting member 83 and the clamp member 84, and the distal end surface of the proximal end outward flange of the lens frame 74. Therefore, a clamp mechanism clamping the electrical cables 78 is formed, and a part clamped by the clamp mechanism in each electrical cable 78 is referred to as a clamp portion 86.

A small diameter portion 87, a taper portion 88 and a large diameter portion 89 are formed from the proximal end side to the distal end side by the outer peripheral portion of the relay lens portion 68 of the imaging unit 66. The outside diameter is increased from the proximal end side to the distal end side in the taper portion 88 as a winding portion receiving portion and a diameter increasing portion, and the outside diameter of the large diameter portion 89 is larger than that of the small diameter portion 87. As is mentioned above, the proximal end side part of the supporting portion 47 of the imaging housing 46, the pushing member 53 and the covering portion 42 of the operation coupling member 39 define the proximal end side coupling space 63 having the comparatively large outside diameter, and the small diameter portion 87 is arranged in the proximal end side coupling space 63. A circular cylinder-shaped housing space 91 wide in the radial direction is formed between the proximal end side part of the supporting portion 47 and the pushing member 53, and the small diameter portion 87. The electrical cable 78 extended from the clamp mechanism is loosely wound around the small diameter portion 87 as a wound portion to form a winding portion forming a play in the housing space 91. That is, the electrical cable 78 has additional length forming the winding portion 92. It would be noted that, in the present application, a state of winding around a wound portion includes states from a state of directly winding around the wound portion to a state of forming a shape of a very gentle circular arc about the wound portion. Furthermore, each of the central bores of the covering portion 42 of the operation coupling member 39, the support member 38 and the proximal end wall 37 of the operation housing 36 form a distal end side coupling space 62 having the comparatively small outside diameter, and the taper portion 88 and the large diameter portion 89 are arranged in the distal end side coupling space 62. An only slight radial gap is formed between the support member 38 and the proximal end wall 37, and the large diameter portion 89.

The covering portion 42 of the operation coupling member 39, the support member 38 and the proximal end wall 37 of the operation portion housing 36 forms a division wall. An insertion bore 93 passes through the division wall in the axial direction as well as the distal end side coupling space 62 through which the imaging unit 66 is inserted. The wound electrical cables 78 are inserted through the insertion bore 93 and inserted into the operation portion 21. The electrical cables 78 inserted into the operation portion 21 are connected to the operation switches 34 provided in the operation portion main portion 24.

In the embodiment, a plurality of electrical cables 78 are used, and the electrical cables 78 extend so as to be arranged side by side.

Hereinafter, action of the electrical cable 78 will be explained.

Since the endoscope is carried and used, the winding portion 92 forming the play in the electrical cable 78 may be urged toward the operation portion 21 due to influence of gravity and the like. Since the winding portion 92 is formed by winding the electrical cable 78 around the small diameter portion 87 and hard to be moved in the axial direction, movement of the winding portion 92 toward the operation portion 21 is restricted. Moreover, even when the winding portion 92 is moved toward the operation portion 21, the taper portion 88 supports the winding portion 92, and movement of the winding portion 92 toward the operation portion 21 beyond the taper portion 88 is restricted. Thus, movement of the winding portion 92 forming the play in the electrical cable 78 into the operation space 61 is restricted, and the part forming the play in the electrical cable 78 is prevent from being brought into contact with the image guide fiber 26.

Moreover, when the monitor portion 27 is rotated about the rotational axis O relative to the operation portion 21, the circuit board 79 and the imaging unit 66 are rotated about the rotational axis O together with the imaging housing 46 of the imaging assembly 28. A part from the connecting end portion 82 to the clamp portion 86 in the electrical cable 78 is rotated together with the electric contact portion 81 of the circuit board 79 and the clamp mechanism of the imaging unit 66. Since the electrical cable 78 has the sufficient additional length taking a form of the winding portion 92, the connecting end portion 82 of the electrical cable 78 does not pull the electric contact portion 81 of the circuit board 79 to prevent rotation of the imaging assembly 28. That is, when the monitor portion 27 is rotated in one direction relative to the operation portion 21, the proximal end portion of the winding portion 92 of the electrical cable 78 is pulled in the rotational direction, and the winding portion 92 is acted so as to be wound around the small diameter portion 87 of the imaging unit 66. On the other hand, when the monitor portion 27 is rotated in the opposite direction relative to the operation portion 21, the proximal end portion of the winding portion 92 of the electrical cable 78 is pushed forward in the rotational direction, and the winding portion 92 is acted so as to be released from the small diameter portion 87 of the imaging unit 66. Thus, since the winding portion 92 forming the play is acted regularly in the radial direction, action of the winding portion 92 toward the operation portion 21 resulting from irregular action is restricted. Moreover, even when the winding portion 92 is acted toward the operation portion 21, the taper portion 88 supports the winding portion 92, and action of the winding portion 92 toward the operation portion 21 beyond the taper portion 88 is restricted. Thus, even when the monitor portion 27 is rotated relative to the operation portion 21 and the winding portion 92 forming the play in the electrical cable 78 is acted following the monitor portion 27, action of the winding portion 92 into the operation space 61 is restricted, and the part forming the play in the electrical cable 78 is prevented from being brought into contact with the image guide fiber 26.

As is mentioned above, in the endoscope according to the embodiment, the part forming the play in the electrical cable 78 is wound around the small diameter portion 87 of the imaging unit 66 to form the winding portion 92, and thus, movement of the part forming the play toward the operation portion 21 is restricted. Moreover, even when the winding portion 92 forming the play is moved toward the operation portion 21, the taper portion 88 supports the winding portion 92, and movement of the winding portion 92 toward the operation portion 21 beyond the taper portion 88 is restricted. Furthermore, since the winding portion 92 is acted regularly, that is, is wound around or released from the small diameter portion 87 when the monitor portion 27 is rotated relative to the operation portion 21 and the winding portion 92 is acted following the monitor portion 27, action of the part forming the play toward the operation portion 21 resulting from irregular action is restricted. Moreover, even when the winding portion 92 is acted irregularly toward the operation portion 21, the taper portion 88 supports the winding portion 92, and action of the winding portion 92 toward the operation portion 21 beyond the taper portion 88 is restricted. Thus, contact of the part forming the play in the electrical cable 78 and the image guide fiber 26 with each other is certainly avoided, and the image guide fiber 26 is sufficiently prevented from being damaged.

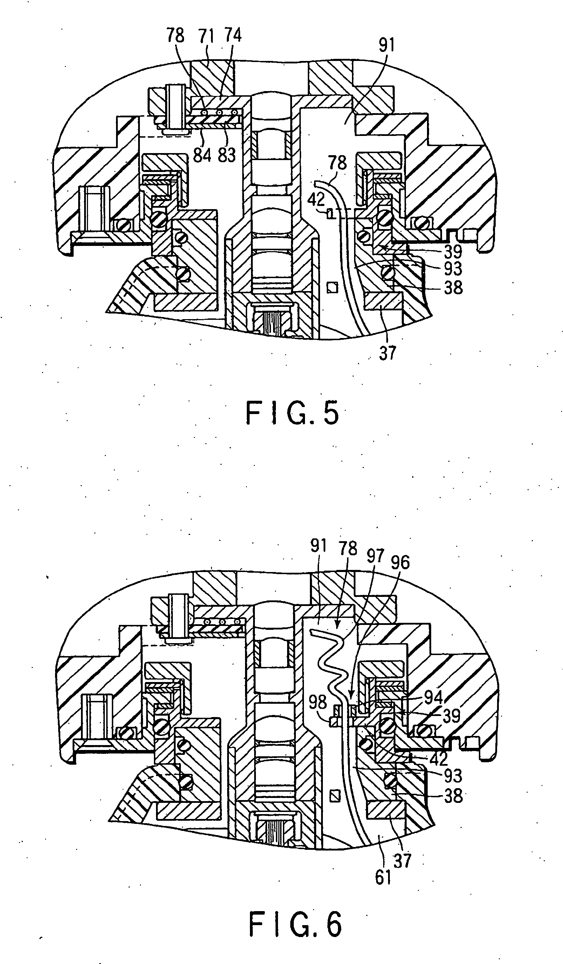

Referring to FIG. 6, a second embodiment of the present invention will be explained.

In the embodiment, regarding each of electrical cables 78, a cylinder-shaped diameter expanding member 94 is provided on the outside of and fixed to the electrical cable 78, and a diameter expanding portion 96 as a restricting portion is formed in the electrical cable 78, which expands the outside diameter. Moreover, a part between the diameter expanding portion 96 and a connecting end portion 82 forms a play portion 97 forming a play in the electrical cable 78. The play portion 97 and the diameter expanding portion 96 are arranged in a housing space 91. As is mentioned above, an insertion bore 93 is formed at a covering portion 42 of an operation coupling member 39, a support member 38 and a proximal end wall 37 of an operation housing 36 and the electrical cable 78 is inserted through the insertion bore 93, and the proximal end opening of the insertion bore 93 is formed at the proximal end surface of the covering portion 42. The minimum outside diameter of the diameter expanding portion 96 of the electrical cable 78 is larger than the minimum outside diameter of the proximal end opening of the insertion bore 93, and a contact receiving portion 98 is formed by the proximal end surface of the covering portion 42 as a restricting receiving portion and a diameter expanding portion receiving portion and configured to be brought into contact with the diameter expanding portion 96. The diameter expanding portion 96 is brought into contact with the contact receiving portion 98, and thus, movement of the diameter expanding portion 96 toward an operation space 61 is restricted, and movement of the play portion 97 between the diameter expanding portion 96 and the connecting end portion 82 toward the operation space 61 is restricted. Moreover, as is similar to the first embodiment, a taper portion 8.8 as a play portion receiving portion restricts movement of the play portion 97 toward an operation portion 21, and action of the play portion 97 toward the operation portion 21 is restricted during rotational operation of a monitor portion 27. Therefore, contact of the play portion 97 of the electrical cable 78 with an image guide fiber 26 is certainly avoided, and the image guide fiber 26 is sufficiently prevented from being damaged.

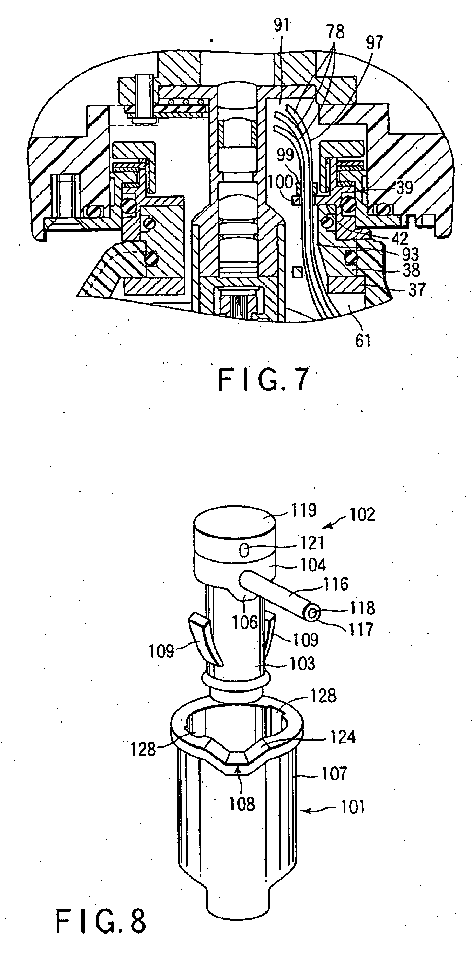

Referring to FIG. 7, a third embodiment of the present invention will be explained.

In the embodiment, a binding portion 99 is formed by binding electrical cables 78. Regarding each of the electrical cables 78, a play portion 97 forms a play is formed between the binding portion 99 and a connecting end portion 82 in the electrical cable 78. The play portion 97 and the binding portion 99 are arranged in a housing space 91. The minimum outside diameter of the binding portion 99 is larger than the minimum outside diameter of an,insertion bore 93 of a covering portion 42, and a support receiving portion 100 as a restricting receiving portion and a binding portion receiving portion is formed by the edge part defining the proximal end opening of the insertion bore 93 in the covering portion 42 and is configured to support the binding portion 99. The support receiving portion 100 supports the binding portion 99, and thus, movement of the binding portion 99 toward an operation space 61 is restricted, and movement of the play portion 97 between the binding portion 99 and the connecting end portion 82 toward the operation space 61 is restricted. Moreover, as is similar to the second embodiment, a taper portion 88 restricts movement of the play portion 97 toward the operation portion 21, and action of the play portion 97 toward the operation portion 21 is restricted during rotational operation of a monitor portion 27. Therefore, contact of the play portion 97 of the electrical cable 78 with an image guide fiber 26 is certainly avoided, and the image guide fiber 26 is sufficiently prevented from being damaged.

Referring to FIGS. 1 and 8 to 12B, a first referential embodiment of the present invention will be explained.

Referring to FIG. 1, a suction opening is formed at a distal end rigid portion 22 of an insertion portion 20. The distal end portion of an inner tube for suction is coupled to the inner end portion of the suction opening, the inner tube is inserted through the insertion portion 20 and an operation portion 21, and the proximal end portion of the inner tube is coupled to the inner end portion of a suction cylinder 101 of the operation portion 21. A suction valve 102 is configured to be attached to and detached from the suction cylinder 101 and is provided in the suction cylinder 101. Hereinafter, the suction cylinder 101 and the suction valve 102 are referred to as a valve unit together with each other. One end portion of an outer tube for suction is connected to the suction valve 102, and the other end portion of the outer tube is connected to a suction apparatus. The suction opening, the inner tube, the valve unit and the outer tube forms a suction channel, and the suction apparatus is configured to suck from the suction opening via the suction channel.

Since treated tissue, blood, dirt and so forth are sucked via the suction channel, the suction channel tends to become dirt. Therefore, it is necessary, in particular, to sufficiently clean the suction channel when cleaning an endoscope. When cleaning the endoscope, in order to improve cleaning efficiency, the suction valve 102 is detached, and then, the cleaning is performed. Furthermore, in the referential embodiment, the suction valve 102 is disposable and cleaning work for the suction valve 102 is omitted, and thus, cleaning work becomes easy.

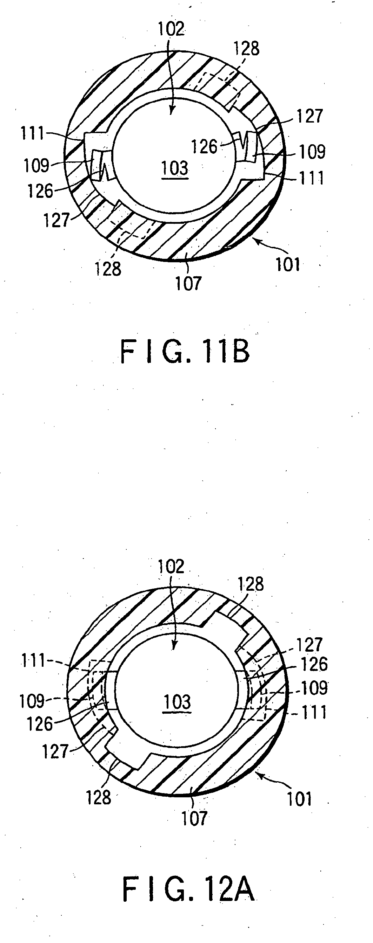

Referring to FIGS. 8 to 12B, a structure of the valve unit will be explained in detail.

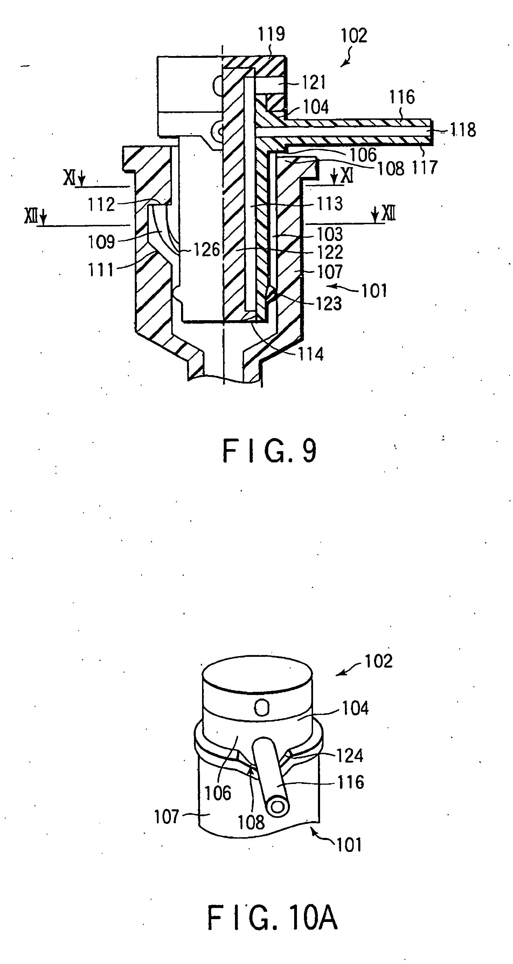

Referring to FIGS. 8 and 9, an attachment mechanism will be explained, which is configured to attach the suction valve 102 to the suction cylinder 101 in the valve unit.

The suction valve 102 includes a circular cylinder-shaped attachment portion 103. A short circular cylinder-shaped middle coupling portion 104 coaxially covers the axially outer end portion of the attachment portion 103. The middle coupling portion 104 overhangs outwardly in the radial direction beyond the attachment portion 103, and a convex portion 106 inwardly in the axial direction is formed at the axially inner end surface of the overhanging part. The convex portion 106 is in the shape of a trapezoid whose base is arranged on the axially outer side when viewing in the radial direction. On the other hand, a circular cylinder-shaped attachment receiving portion 107 is formed at the suction cylinder 101, and the attachment portion 103 of the suction valve 102 is configured to be inserted into and pulled out from the attachment receiving portion 107. A concave portion 108 inwardly in the axial direction is formed at the axially outer end surface portion of the suction cylinder 101. The concave portion 108 is in the shape of a trapezoid whose base is arranged on the axially outer side when viewing in the radial direction and which corresponds to the convex portion 106. The convex portion 106 is configured to be fitted on the concave portion 108 to restrict rotation of the suction valve 102 relative to the suction cylinder 101 when the attachment portion 103 is inserted into the attachment receiving portion 107.

An elastic plate-shaped claw portion 109 is provided at the axially middle portion of the outer peripheral surface of the attachment portion 103. The root portion of the claw portion 109 is coupled to the outer peripheral surface of the attachment portion 103, and the terminal end side part of the claw portion 109 faces the outer peripheral surface of the attachment portion 103 and opens outwardly in the radial direction from the outer peripheral surface of the attachment portion 103 by a certain angle. The claw portion 109 is configured to be closed inwardly in the radial direction to the closing position by pushing force inwardly in the radial direction of the inner peripheral surface of the attachment receiving portion 107 and slide on the inner peripheral surface of the attachment receiving portion 107 when the attachment portion 103 is inserted into the attachment receiving portion 107. A claw receiving portion 111 is formed at the axially middle portion of the inner peripheral portion of the attachment receiving portion 107, and the claw receiving portion 111 has a concave shape outwardly in the radial direction. The claw portion 109 is configured to be returned outwardly in the radial direction to the natural position and housed within the claw receiving portion 111 when the attachment portion 103 is inserted into the attachment receiving portion 107 and the claw portion 109 is inserted to the claw receiving portion 111. An engagement receiving surface 112 is formed at the axially outer end wall of the claw receiving portion 111. The terminal end portion of the claw portion 109 is brought into contact with and engaged with the engagement receiving surface 112 when the claw portion 109 is housed within the claw receiving portion 111. Movement of the suction valve 102 outwardly in the axial direction relative to the suction cylinder 101 is restricted when the engagement receiving surface 112 and the terminal end portion of the claw portion 109 are brought into contact and engaged with each other.

The position of the suction valve 102 relative to the suction cylinder 101 when the suction valve 102 is attached to the suction cylinder 101 is referred to as an attachment position.

Referring to FIGS. 8 and 9, a valve mechanism in the valve unit will be explained.

A suction pass 113 is formed within the attachment portion 103 of the suction valve 102. An inlet opening 114 is formed at the proximal end surface of the attachment portion 103 and is in fluid communication with the suction pass 113. An arm portion 116 protrudes from the outer peripheral surface of the middle coupling portion 104. A suction connecter 117 is formed at the protruding end portion of the arm portion 116 and is configured to be connected with the outer tube for suction. An outlet opening 118 is formed at the suction connecter 117 and is in fluid communication with the suction pass 113. A suction button 119 covers the axially outer end portion of the middle coupling portion 104. The suction button 119 is configured to be deformed to be compressed in the axial direction to an operation position by pushing operation, and is configured to be deformed to be returned in the axial direction to the normal position due to its elasticity by releasing the pushing operation. A leak opening 121 is formed at the suction button 119 and is in fluid communication with the suction pass 113, and the suction button 119 is coupled to a piston body 122 arranged slidably within the suction pass 113. When the suction button 119 is in the normal position, the piston body 122 closes the inlet opening 114, the leak opening 121 is opened, and only the leak opening 121 and the outlet opening 118 are in fluid communication with each other. Therefore, no suction is performed from the inlet opening 114 even when sucking from the outlet opening 118. On the other hand, when the suction button 119 is in the operation position, the piston body 122 opens the inlet opening 114, the leak opening 121 is closed by the compressive deformation of the suction button 119, and only the inlet opening 114 and the outlet opening 118 are in fluid communication with each other. Therefore, suction is preformed from the inlet opening 114 when sucking from the outlet opening 118.

A sealing portion 123 is formed at the axially inner end portion of the outer peripheral surface of the attachment portion 103. The sealing portion 123 has elasticity, protrudes outwardly in the radial direction and extends over the whole periphery. The sealing portion 123 is configured to be stuck on and engaged with the inner peripheral surface of the attachment receiving portion 107 when the attachment portion 103 is inserted into the attachment receiving portion 107. A space on the axially inside of the sealing portion 123 is configured to be sealed from the outside when the inner peripheral surface of the attachment receiving portion 107 and the sealing portion 123 are stuck on and engaged with each other.

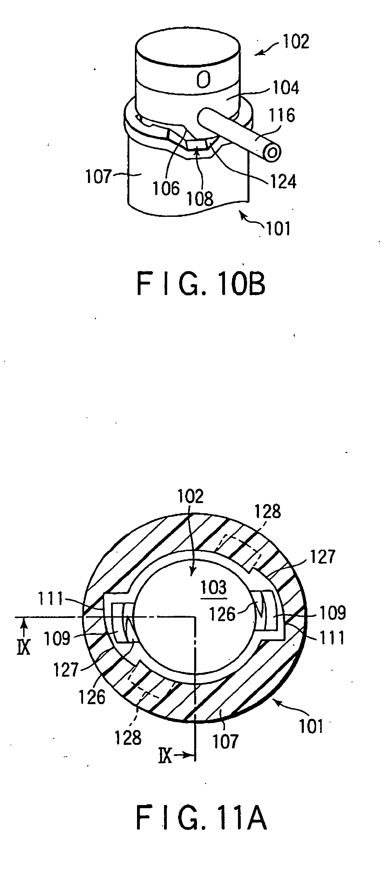

Referring to FIGS. 8 to 11B, a breaking mechanism will be explained, which is configured to break the claw portion 109.

The suction valve 102 is configured to be rotated from the attachment position (referring to FIGS. 10A and 11A) to the detachment position (referring to FIGS. 10B and 11B) relative to the suction cylinder 101 by operating the arm portion 116 to be rotated. Hereinafter, the above rotational direction is referred to as an operation direction.

A cam surface 124 is formed by both the side surfaces of the concave portion 108 at the attachment receiving portion 107 of the suction cylinder 101. On the other hand, the convex portion 106 of the attachment portion 103 of the suction valve 102 functions as a cam. the suction valve 102 is configured to be moved in a pulling-out direction relative to the suction cylinder 101 due to an interaction between the cam surface 124 of the concave portion 108 and the convex portion 106 when the suction valve 102 is rotated relative to the suction cylinder 101.

A fragile thin portion 126 is formed at a part on the side in the operation direction in the root portion of the claw portion 109 of the attachment portion 103. On the other hand, a gradually changing portion 127 is formed at the axially middle part of the inner peripheral portion of the attachment receiving portion 107 and has a concave shape outwardly in the radial direction. The gradually changing portion 127 is arranged on the side in the operation direction of the claw receiving portion 111, is continued with the claw receiving portion 111 and extends in the operation direction. The inside diameter of the gradually changing portion 127 gradually decreases in the operation direction. The side end portion of the claw portion 109 is configured to be hooked on the inner peripheral surface of the gradually changing portion 127 to apply tearing force to the thin portion 126 to break the thin portion 126 when the suction valve 102 is rotated in the operation direction from the attachment position relative to the suction cylinder 101 and the claw portion 109 housed within the claw receiving portion 111 is rotated in the operation direction. The axially outer end wall of the gradually changing portion 127 is inclined outwardly in the axial direction toward the operation direction so as not to interfere with movement of the claw portion 109 outwardly in the axial direction along with movement of the suction valve 102 outwardly in the axial direction. When the claw portion 109 is arranged at the side end portion in the operation direction of the gradually changing portion 127, the suction valve 102 is in the detachment position relative to the suction cylinder 101.

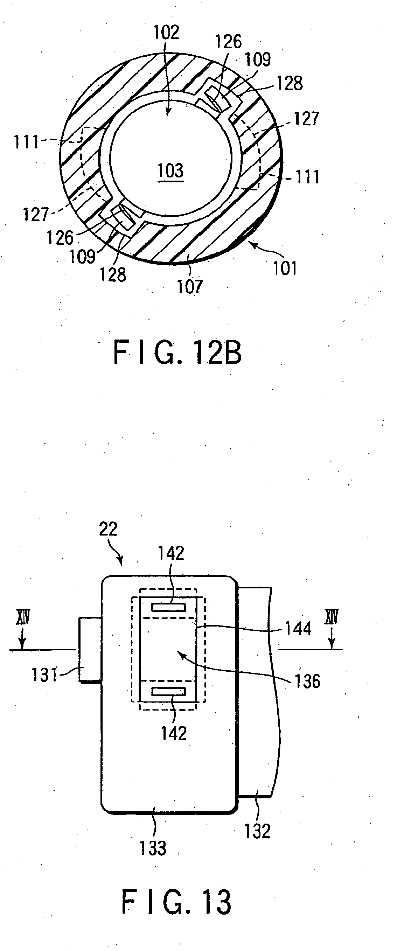

Referring to FIGS. 8, 12A and 12B, a detachment mechanism will be explained, which is configured to detach the suction valve 102 from the suction cylinder 101.

A detachment groove 128 is formed at the axially outer side part of the inner peripheral portion of the attachment receiving portion 107. The detachment groove 128 is arranged on the axially outer side of the side end portion in the operation direction of the gradually changing portion 127, is continued with the gradually changing portion 127 and extends in the axial direction to the outer end of the attachment portion 103. The claw portion 109 is configured to be aligned with the detachment groove 128 in the axial direction when the suction valve 102 is rotated from the attachment position (referring to FIG. 12A) to the detachment position (referring to FIG. 12B) relative to the suction cylinder 101. The claw portion 109 is configured to slide outwardly in the axial direction in the detachment groove 128 when the suction valve 102 is pulled out from the suction cylinder 101.

Hereinafter, a method for using the valve unit will be explained.

When using the endoscope, the suction valve 102 is attached to the suction cylinder 101. That is, the attachment portion 103 of the suction valve 102 is inserted into the attachment receiving portion 107 of the suction cylinder 101. When inserting the suction valve 102, the claw portion 109 is pushed by the inner peripheral surface of the attachment receiving portion 107 to be closed inwardly in the radial direction to the closing position, and the claw portion 109 slides on the inner peripheral surface of the attachment portion 103. When the claw portion 109 is inserted to the claw receiving portion 111, the claw portion 109 is returned outwardly in the radial direction to the natural position, the claw portion 109 is housed within the claw receiving portion 111, and the terminal end portion of the claw portion 109 is brought into contact with and engaged with the engagement receiving surface 112 of the claw receiving portion 111. Moreover, the convex portion 106 of the suction valve 102 is fitted on the concave portion 108 of the suction cylinder 101. The engagement receiving surface 112 and the terminal end portion of the claw portion 109 are brought into contact with and engaged with each other, and thus, movement of the suction valve 102 outwardly in the axial direction relative to the suction cylinder 101 is restricted. Moreover, the concave portion 108 and the convex portion 106 are fitted on each other, and thus, rotation of the suction valve 102 relative to the suction cylinder 101 is restricted.

Next, the suction valve 102 is connected with the outer tube and the suction apparatus, and the suction apparatus is actuated. When the suction button 119 is not operated to be pushed, air is sucked from the leak opening 121 of the suction button 119. On the other hand, when the suction button 119 is operated to be pushed, suction is performed from the suction opening of the distal end rigid portion 22 of the endoscope.

After using the endoscope, the suction valve 102 is detached from the suction cylinder 101. That is, at first, the arm portion 116 is operated to be rotated, and the suction valve 102 is rotated from the attachment position to the detachment position relative to the suction cylinder 101. The cam surface 124 of the concave portion 108 of the suction cylinder 101 and the convex portion 106 of the suction valve 102 are interacted with each other, and thus, the suction valve 102 is moved outwardly in the axial direction. When the suction valve 102 is rotated, the claw portion 109 of the suction valve 102 is also rotated within the gradually changing portion 127 of the suction cylinder 101, the side end portion of the claw portion 109 is hooked on the inner peripheral surface of the gradually changing portion 127, and the claw portion 109 is rotated, and thus, tearing force is applied to the thin portion 126 and the thin portion 126 is broken. Next, the suction valve 102 is pulled out from the suction cylinder. When the suction valve 102 is pulled out, the claw portion 109 of the suction valve 102 slides within the detachment groove 128 of the suction cylinder. After detaching the suction valve 102, the suction valve 102 is appropriately disposed.

In the valve unit according to the referential embodiment, in an attachment of the suction valve 102 to the suction cylinder 101, when the attachment portion 103 of the suction valve 102 is inserted into the attachment receiving portion 107 of the suction cylinder 101, the inner peripheral surface of the attachment receiving portion 107 closes the claw portion 109 of the attachment portion 103 inwardly in the radial direction to the closing position, and the claw portion 109 slides on the inner peripheral surface of the attachment receiving portion 107. Thus, the claw portion 109 does not prevent the attachment portion 103 from inserting into the attachment receiving portion 107, and attachment efficiency of the suction valve 102 to the suction cylinder 101 is improved.

Moreover, when the suction valve 102 is attached to the suction cylinder 101, the claw portion 109 of the attachment portion 103 of the suction valve 102 is housed within the claw receiving portion 111 of the attachment receiving portion 107 of the suction cylinder 101. The terminal end portion of the claw portion 109 is brought into contact with and engaged with the engagement receiving surface 112 of the claw receiving portion 111. Therefore, when using the endoscope, pulling out of the attachment portion 103 from the attachment receiving portion 107 is restricted, and thus, the suction valve 102 is prevented from being unintentionally detached from the suction cylinder 101.

Furthermore, when detaching the suction valve 102 from the suction cylinder 101, it is necessary to operate the suction valve 102 to be rotated from the attachment position to the detachment position relative to the suction cylinder 101 before a detachment, and the claw portion 109 of the suction valve 102 is broken by the rotational operation. Therefore, the claw portion 109 of the suction valve 102 is broken after detaching the suction valve 102, and thus, the disposable suction valve 102 is prevented from being used again.

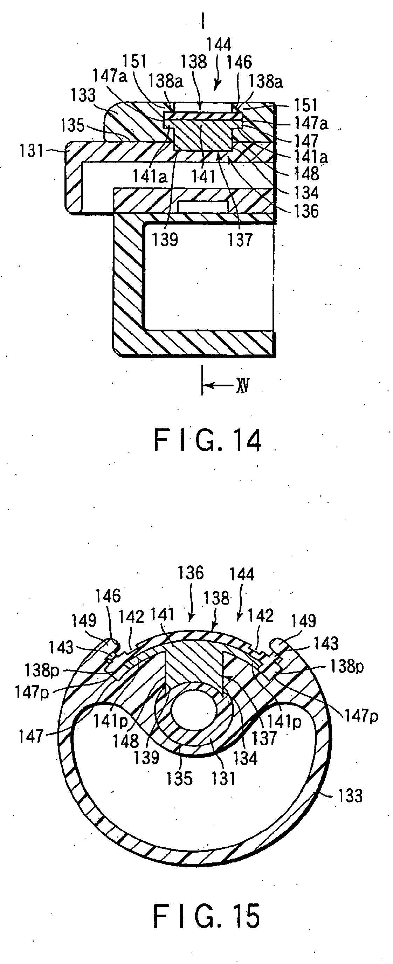

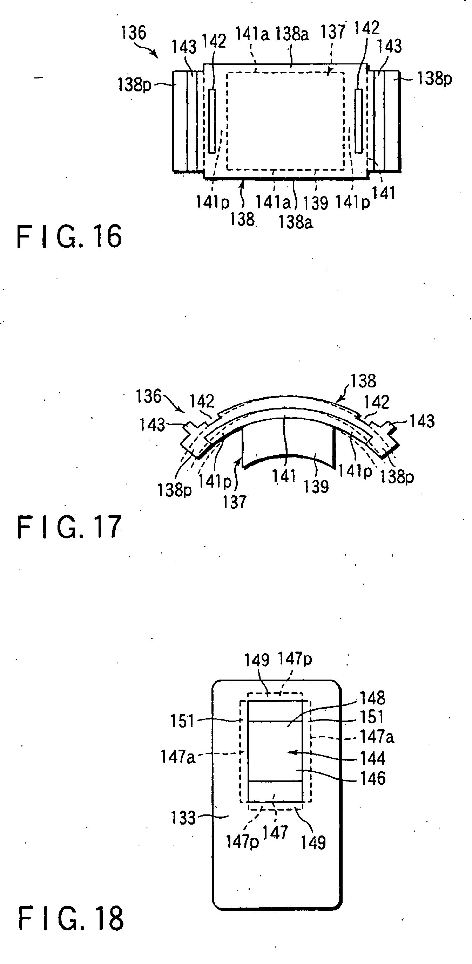

Referring to FIGS. 13 to 18, a second referential embodiment of the present invention will be explained.

Referring to FIG. 13, an observation optical system is built-in a distal end rigid portion 22 of an endoscope, and the distal end surface of the most distal optical element of the observation optical system is exposed at the distal end surface of the distal end rigid portion 22. Moreover, the distal end portion of a nozzle 131 protrudes from the distal end surface portion of the distal end rigid portion 22, and the distal end portion of the nozzle 131 is directed toward the exposed surface of the optical element. It is possible to remove dirt adhered to the exposed surface of the optical element by sending liquid or gas to the exposed surface.

Hereinafter, a fixing mechanism for the nozzle 131 in the distal end rigid portion 22 will be explained in details.

Referring to FIGS. 13 to 15, a distal cover member 133 made of resin covers the distal end side of a distal end main portion member 132 made of metal in the distal end rigid portion 22. An attachment bore 135 passes through the distal cover member 113 in the axial direction. The nozzle 131 is in the shape of a hook, the distal end part of the nozzle 131 protrudes from the distal end surface of the distal cover member 113, and the proximal end side part of the nozzle 131 is fitted into the attachment bore 135 of the distal cover member 113. A fixing concave portion 134 is formed at a part in the outer peripheral portion of the proximal end side part of the nozzle 131, which is arranged on the radially outer side relative to the central axis of the distal end rigid portion 22.

Referring to FIGS. 13 to 17, a nozzle holding unit 136 will be explained.

A sticking member 138 is overlapped with and fixed to the radially outer side of a fixing member 137 in the nozzle holding unit 136.

The fixing member 137 is made of metal, for example, stainless steel. A fixing portion 139 and an urging portion 141 are integrally formed from the radially inner side to the radially outer side in the fixing member 137. The fixing portion 139 is in the shape of a thick bending plate, has a rectangular shape when viewing in the radial direction and is in the shape of a circular arc about the central axis of the distal end rigid portion 22 when viewing in the axial direction. The urging portion 141 is in the shape of a thin bending plate and includes a central part, peripheral overhanging portions 141p and axial overhanging portions 141a. When viewing in the radial direction, the urging portion 141 has a rectangular shape, each part has a rectangular shape, the central part has the same shape as that of and overlapped with the fixing portion 139, the peripheral overhanging portions 141p overhang in the peripheral directions from the central part, and the axial overhanging portions 141a overhang a little in the axial directions from the central part and the peripheral overhanging portions 141p. Moreover, when viewing in the axial direction, the central part and the axial overhanging portions 141a are in the shape of a circular arc about the central axis of the distal end rigid portion 22, and the peripheral overhanging portions 141p is in the shape of a circular arc having the radius of curvature larger than that of a circular arc about the central axis of the distal end rigid portion 22 and arranged on the radially outer side relative to the central part and the axial overhanging portions 141a.

The sticking member 138 is made of elastic material having biocompatibility, chemical-resistance and heat-resistance, for example, fluoro rubber. The sticking member 138 is in the shape of a thin bending plate, includes a central part, peripheral overhanging portions 138p and axial overhanging portions 138a. When viewing in the radial direction, the sticking member 138 is in the shape of a cross, each part has a rectangular shape, the central part has the same shape as that of and overlapped with the central part and the peripheral overhanging portions 141p of the urging portion 141, the peripheral overhanging portions 138p overhang in the peripheral directions from the central part, and the axial overhanging portions 138a overhang a little in the axial directions from the central part and has the same shape as that of and overlapped with the axial overhanging portions 141a of the urging portion 141. When viewing in the axial direction, the sticking member 138 is in the shape of a circular arc along the urging portion 141. Furthermore, picking grooves 142 are formed at the peripherally end portions of the outer peripheral portion in the central part of the sticking member 138, are recessed inwardly in the radial direction and extend in the axial direction. Moreover, projecting portions 143 are formed at the outer peripheral portion in the peripheral overhanging portions 138p of the sticking member 138, protrude outwardly in the radial direction and extend over the total length in the axial direction.

Referring to FIGS. 13 to 15 and 18, a fixing opening 144 of the distal cover member 113 will be explained.

The fixing opening 144 is formed at the radially outer side of the attachment bore 135 in the distal cover member 113. An attachment and detachment opening portion 146, a holding opening portion 147 and an insertion opening portion 148 are formed from the radially outer side to the radially inner side in the fixing opening 144. The attachment and detachment opening portion 146 forms a space in the shape of a thin bending plate, has a rectangular shape when viewing in the radial direction, and is in the shape of a circular arc about the central axis of the distal end rigid portion 22 when viewing in the axial direction. The holding opening portion 147 forms a space in the shape of a thin bending plate and includes a central part, peripheral overhanging portions 147p and axial overhanging portions 147a. When viewing in the radial direction, the holding opening portion 147 is in a shape of a cross, each part has a rectangular shape, the central part has the same shape as that of and overlapped with the attachment and detachment opening portion 146, the peripheral overhanging portions 147p overhang in the peripheral directions from the central part, and the axial overhanging portions 147a overhang a little in the axial directions from the central part. When viewing in the axial direction, the holding opening portion 147 is in the shape of a circular arc about the central axis of the distal end rigid portion 22. The part defining the peripheral overhanging portions 147p of the holding opening portion 147 are referred to as axial holding portions 149, and the part defining the axial overhanging portions 147a are referred to as peripheral holding portions 151 in the distal cover member 113. The insertion opening portion 148 forms a space in the shape of a thick bending plate, has a rectangular shape when viewing in the radial direction and is in the shape of a circular arc about the central axis of the distal end rigid portion 22 when viewing in the axial direction. The insertion opening portion 148 is concentric with the central part of the holding opening portion 147 when viewing in the radial direction, and has the same axial width as that of and a smaller peripheral length than that of the central part of the holding opening portion 147. Moreover, the insertion opening portion 148 is connected to the attachment bore 135 of the nozzle 131.

Referring to FIGS. 13 to 18, a fixing mechanism for the nozzle 131 will be explained in details.

The urging portion 141 of the fixing member 137 and the sticking member 138 of the nozzle holding unit 136 are arranged within the holding opening portion 147 of the fixing opening 144. The peripheral overhanging portions 147p of the holding opening portion 147 are defined by the axial holding portions 149, and the peripheral overhanging portions 138p of the sticking member 138 are fitted in the peripheral directions into the axial holding portions 149 in the distal cover member 113. Since the radius of curvature of the peripheral overhanging portions 141p of the urging portion 141 is larger than that of the inner peripheral wall of the axial holding portions 149 in the natural state, the peripheral overhanging portions of the sticking member 138 are urged against the inner peripheral walls of the axial holding portions 149 by repulsive force produced by the urging portion 141, and the projecting portions 143 of the peripheral overhanging portions 138p of the sticking member 138 are stuck to and engaged with the inner peripheral walls. Therefore, sealing is formed between the nozzle holding unit 136 and the distal cover member 113 along the axial direction. Moreover, the axial overhanging portions 147a of the holding opening portion 147 are defined by the peripheral holding portions 151, and the axial overhanging portions 141a and 138a of the urging portion 141 and the sticking member 138 are fitted in the axial directions into the peripheral holding portions 151 in the distal cover member 113. The axial width of the axial overhanging portions 141a and 138a of the urging portion 141 and the sticking member 138 is slightly larger than that of the peripheral holding portions 151, and the sticking member 138 is deformed to be compressed by and brought into contact and engaged with the inner walls of the peripheral holding portions 151. Therefore, sealing is formed between the nozzle holding unit 136 and the distal cover member 113 along the peripheral direction. Moreover, the fixing portion 139 of the fixing member 137 of the nozzle holding unit 136 is fitted in the radial direction into the insertion opening portion 148 of the fixing opening 144, and the fixing portion 139 is fitted into the fixing concave portion 134 of the nozzle 131 within the attachment bore 135. Therefore, the nozzle 131 is fixed relative to the distal cover member 113. The picking grooves 142 of the central part of the sticking member 138 are exposed to the outside of the distal cover member 113 through the attachment and detachment opening portion 146.

Hereinafter, a method of attaching and detaching the nozzle 131 will be explained.

When attaching the nozzle 131 to the distal cover member 113, the nozzle 131 is inserted into the attachment bore 135. Next, the pair of picking grooves 142 of the sticking member 138 of the nozzle holding unit 136 is picked by tweezers, and the peripheral overhanging portions 141p of the urging portion 141 of the fixing member 137 are deformed inwardly in the radial direction together with the sticking member 138. Then, the nozzle holding unit 136 is inserted into the fixing opening 144, the peripheral overhanging portions 138p of the sticking member 138 are fitted into the axial holding portions 149 of the distal cover member 113, the axial overhanging portions 138a of the sticking member 138 and the axial overhanging portions 141a of the urging portion 141 are fitted into the peripheral holding portions 151 of the distal cover member 113, the fixing portion 139 of the fixing member 137 is fitted into the insertion opening portion 148 of the fixing opening 144, and the fixing portion 139 is fitted into the fixing concave portion 134 of the nozzle 131 within the attachment bore 135. Thus, the nozzle 131 is attached to the distal cover member 113. When detaching the nozzle 131 from the distal cover member 113, the pair of picking grooves 142 is picked by tweezers through the attachment and detachment opening portion 146, the peripheral overhanging portions 141p of the urging portion 141 are deformed inwardly in the radial direction together with the sticking member 138 and simultaneously the whole nozzle holding unit 136 is pulled out from the fixing opening 144, and the fixing portion 139 of the nozzle holding unit 136 is pulled out from the fixing concave portion 134 of the nozzle 131. After that, the nozzle 131 is pulled out from the attachment bore 135. Thus, the nozzle 131 is detached from the distal cover member 113.

In the fixing mechanism of the nozzle 131 according to the referential embodiment, the nozzle 131 is enabled to be fixed to and pulled out from the distal cover member 113 just by inserting and pulling out the nozzle holding unit 136 into and from the fixing opening 144, and also, it is unnecessary to exchange the distal cover member 113 when exchanging the nozzle 131. Therefore, it is very easy to exchange the nozzle 131.

## Claims

1. An endoscope comprising:
an operation portion (21) including an inner space (61);
an optical image conducting member (26) configured to conduct an imaged optical image and including a coupling end portion arranged in the inner space (61) of the operation portion (21);
a display portion (27) coupled to the operation portion (21) configured to be rotated about a central axis as a rotational axis of an elongated insertion portion (20) of the endoscope relative to the operation portion (21), including an inner space (64) and configured to display the imaged optical image conducted by the optical image conducting member (26); and
an electrical cable (78) configured to transmit an electric signal, extending between the inner space (61) of the operation portion (21) and the inner space (64) of the display portion (27), including a connecting end portion (82) connected to the display portion (27) and including a winding portion (92) formed by additional length so as not to prevent movement of the display portion (27) relative to the operation portion (21),
**characterized by**
a wound portion (87) provided in the inner space (61) of the operation portion (21) or the inner space (64) of the display portion (27), and including a diameter increasing portion (88) configured to increase from a proximal end side to a distal end side along the rotational axis, wherein
the winding portion (92) is configured to be wound around the wound portion (87) and the diameter increasing portion (88) is configured to support the winding portion (92) such that movement of the winding portion (92) toward the operation portion (21) beyond the diameter increasing portion (88) is restricted, and
the winding portion (92) is configured such that when the display portion (27) is rotated in a first direction relative to the operation portion (21), a proximal end portion of the winding portion (92) is pulled in the rotational direction, and the winding portion (92) is acted so as to be wound around the wound portion (87), and when the display portion (27) is rotated in a second direction relative to the operation portion (21), which is opposite to the first direction, the proximal end portion of the winding portion (92) is pushed forward in the rotational direction, and the winding portion (92) is acted so as to be released from the wound portion (87).

2. The endoscope according to claim 1, **characterized in that**
the display portion (27) includes an imaging unit (66) configured to image the optical image conducted by the optical image conducting member (26),
the imaging unit (66) is arranged along the central axis of the insertion portion (20) of the endoscope, extends in the inner space (64) of the display portion (27) and protrudes into the inner space (61) of the operation portion (21),
the coupling end portion of the optical image conducting member (26) is coupled to a protruding end portion of the imaging unit (66), and
the wound portion (87) is formed on an outer peripheral portion of the imaging unit (66).

3. The endoscope according to claim 1, **characterized in that**
the display portion (27) includes an imaging unit (66) configured to image the optical image conducted by the optical image conducting member (26),
the imaging unit (66) is arranged along the central axis of the insertion portion (20) of the endoscope, extends in the inner space (64) of the display portion (27) and protrudes into the inner space (61) of the operation portion (21),
the coupling end portion of the optical image conducting member (26) is coupled to a protruding end portion of the imaging unit (66),
the wound portion (87) is formed by a small diameter portion (87) provided at an outer peripheral portion of the imaging unit (66),
the endoscope includes a winding portion receiving portion (88) provided in the inner space (61) of the operation portion (21) or the inner space (64) of the display portion (27), arranged between the winding portion (92) and the optical image conducting member (26) and configured to restrict movement of the winding portion (92) toward the optical image conducting member (26), and
the winding portion receiving portion (88) is formed by the diameter increasing portion (88) provided at the outer peripheral portion of the imaging unit (66), arranged closer to the operation portion (21) than the small diameter portion (87) and has an outer diameter larger than an outer diameter of the small diameter portion (87).

## Patentansprüche

1. Endoskop aufweisend:
ein Betätigungsteil (21) enthaltend einen inneren Raum (61);
ein Element zum Leiten eines optischen Bildes (26), das dazu eingerichtet ist ein abgebildetes optisches Bild zu leiten und ein Kupplungsendteil enthält, das in dem inneren Raum (61) des Betätigungsteils (21) angeordnet ist;
ein Anzeigeteil (27), das mit dem Betätigungsteil (21) gekoppelt und dazu eingerichtet ist, um eine Mittelachse als eine Drehachse eines gestreckten Einführungsteils (20) des Endoskops relativ zu dem Betätigungsteil (21) gedreht zu werden, enthaltend einen inneren Raum (64) und dazu eingerichtet, das abgebildete optische Bild, das durch das Element zum Leiten des optischen Bildes (26) geleitet wurde, anzuzeigen; und
ein elektrisches Kabel (78), das dazu eingerichtet ist, ein elektrisches Signal zu senden, das sich zwischen dem inneren Raum des Betätigungsteils (21) und dem inneren Raum (64) des Anzeigeteils erstreckt, das ein Verbindungsendteil (82), das mit dem Anzeigeteil (27) verbunden ist, enthält, und das ein Windungsteil (92) enthält, das durch zusätzliche Länge gebildet ist, um nicht eine Bewegung des Anzeigeteils (27) relativ zu dem Betätigungsteil (21) zu verhindern,
**gekennzeichnet durch**
ein gewundenes Teil (87), das in dem inneren Raum (61) des Betätigungsteils (21) oder dem inneren Teil (64) des Anzeigeteils (27) bereitgestellt ist, und ein sich im Durchmesser vergrößerndes Teil (88) enthält, das dazu eingerichtet ist, sich von einer proximalen Endseite zu einer distalen Endseite entlang der Drehachse zu vergrößern,
das Windungsteil (92) dazu eingerichtet ist, um das gewundene Teil (87) gewunden zu werden, wobei das sich im Durchmesser vergrößernde Teil (88) dazu eingerichtet ist, das Windungsteil (92) so zu unterstützen, dass Bewegung des Windungsteils (92) zu dem Betätigungsteil (21) über das sich im Durchmesser vergrößernde Teil (88) beschränkt ist, und
das Windungsteil (92) so eingerichtet ist, dass wenn das Anzeigeteil (27) in eine erste Richtung relativ zu dem Betätigungsteil (21) gedreht wird, ein proximales Endteil des Windungsteils (92) in die Drehrichtung gezogen wird, um das Windungsteil (92) so zu betätigen, dass es um das gewundene Teil (87) gewunden wird, und wenn das Anzeigeteil (27) in eine zweite Richtung relativ zu dem Betätigungsteil (21), die entgegengesetzt zu der ersten Richtung ist, gedreht wird, das proximale Endteil des Windungsteil (92) vorwärts in die Drehrichtung gedrückt wird, und das Windungsteil (92) so betätigt wird, dass es von dem gewundenen Teil (87) gelöst wird.

2. Endoskop nach Anspruch 1, **dadurch gekennzeichnet, dass**
das Anzeigeteil (27) eine Abbildungseinheit (66) enthält, die dazu eingerichtet ist, das durch das Element zum Leiten eines optischen Bildes (26) geleitete optische Bild abzubilden,
die Abbildungseinheit (66) entlang der Mittelachse des Einführungsteils (20) des Endoskops angeordnet ist, sich in den inneren Raum (64) des Anzeigeteils (27) erstreckt und in den inneren Raum (61) des Betätigungsteils (21) vorsteht,
das Kupplungsendteil des Elements zum Leiten des optischen Bildes (26) mit einem vorstehenden Endteil der Abbildungseinheit (66) gekoppelt ist und
das gewundene Teil (67) auf einem äußeren Umfangsteil der Abbildungseinheit (66) gebildet ist.

3. Endoskop nach Anspruch 1, **dadurch gekennzeichnet, dass**
das Anzeigeteil (27) eine Abbildungseinheit (66) enthält, die dazu eingerichtet ist, das durch das Element zum Leiten eines optischen Bildes (26) geleitete optische Bild abzubilden,
die Abbildungseinheit (66) entlang der Mittelachse des Einführungsteils (20) des Endoskops angeordnet ist, sich in den inneren Raum (64) des Anzeigeteils (27) erstreckt und in den inneren Raum (61) des Betätigungsteils (21) vorsteht,
das Kupplungsendteil des Elements zum Leiten des optischen Bildes (26) mit einem hervorstehenden Endteil der Abbildungseinheit (66) gekoppelt ist,
das gewundene Teil (67) durch ein Teil mit einem kleinen Durchmesser (87), das an einem äußeren Umfangsteil der Abbildungseinheit (66) bereitgestellt ist, gebildet ist,
das Endoskop ein Windungsteilaufnahmeteil (88) enthält, das in dem inneren Raum (61) des Betätigungsteils (21) oder dem inneren Raum (64) des Anzeigeteils (27) bereitgestellt ist, zwischen dem Windungsteil (92) und dem Element zum Leiten des optischen Bildes (26) angeordnet ist und dazu eingerichtet ist, Bewegung des Windungsteils (92) zu dem Element zum Leiten des optischen Bildes (26) zu beschränken, und
das Windungsteilaufnahmeteil (88) durch das Teil mit sich vergrößerndem Durchmesser (88), das an dem äußeren Umfangsteil der Abbildungseinheit (66) bereitgestellt ist, gebildet wird, näher an dem Betätigungsteil (21) als das Teil mit kleinem Durchmesser (87) angeordnet ist und einen äußeren Durchmesser aufweist, der größer als ein äußerer Durchmesser des Teils mit einem kleinen Durchmesser (87) ist.

## Revendications

1. Endoscope comprenant :
une partie d'actionnement (21) comprenant un espace interne (61) ;
un élément (26) conducteur d'image optique configuré pour conduire une image optique imagée et incluant une partie d'extrémité de couplage agencée dans l'espace interne (61) de la partie d'actionnement (21) ;
une partie d'affichage (27) couplée à la partie d'actionnement (21) configurée pour tourner autour d'un axe central en tant qu'axe de rotation d'une partie d'insertion allongée (20) de l'endoscope par rapport à la partie d'actionnement (21), incluant un espace interne (64) et configurée pour afficher l'image optique imagée conduite par l'élément (26) conducteur d'image optique ; et
un câble électrique (78) configuré pour transmettre un signal électrique, s'étendant entre l'espace interne (61) de la partie d'actionnement (21) et l'espace interne (64) de la partie d'affichage (27), incluant une partie d'extrémité de connexion (82) connectée à la partie d'affichage (27) et incluant une partie d'enroulement (92) formée par une longueur supplémentaire de façon à ne pas empêcher le déplacement de la partie d'affichage (27) par rapport à la partie d'actionnement (21),
**caractérisé par**
une partie enroulée (87) prévue dans l'espace interne (61) de la partie d'actionnement (21) ou l'espace interne (64) de la partie d'affichage (27), et incluant une partie (88) d'augmentation de diamètre configurée pour augmenter d'un côté d'extrémité proximale à un côté d'extrémité distale le long de l'axe de rotation, dans lequel
la partie d'enroulement (92) est configurée pour être enroulée autour de la partie enroulée (87) et la partie (88) d'augmentation de diamètre est configurée pour supporter la partie d'enroulement (92) d'une manière telle que le déplacement de la partie d'enroulement (92) vers la partie d'actionnement (21) au-delà de la partie (88) d'augmentation de diamètre est limité, et
la partie d'enroulement (92) est configurée d'une manière telle que lorsque la partie d'affichage (27) est mise en rotation dans un premier sens par rapport à la partie d'actionnement (21), une partie d'extrémité proximale de la partie d'enroulement (92) est tirée dans le sens de rotation, et la partie d'enroulement (92) est actionnée de façon à être enroulée autour de la partie enroulée (87), et lorsque la partie d'affichage (27) est mise en rotation dans un deuxième sens par rapport à la partie d'actionnement (21), qui est opposé au premier sens, la partie d'extrémité proximale de la partie d'enroulement (92) est poussée vers l'avant dans le sens de rotation, et la partie d'enroulement (92) est actionnée de façon à être libérée de la partie enroulée (87).

2. Endoscope selon la revendication 1, **caractérisé en ce que**
la partie d'affichage (27) comprend une unité d'imagerie (66) configurée pour imager l'image optique conduite par l'élément (26) conducteur d'image optique,
l'unité d'imagerie (66) est agencée le long de l'axe central de la partie d'insertion (20) de l'endoscope, s'étend dans l'espace interne (64) de la partie d'affichage (27) et fait saillie dans l'espace interne (61) de la partie d'actionnement (21),
la partie d'extrémité de couplage de l'élément (26) conducteur d'image optique est couplée à une partie d'extrémité saillante de l'unité d'imagerie (66), et
la partie enroulée (87) est formée sur une partie périphérique externe de l'unité d'imagerie (66).

3. Endoscope selon la revendication 1, **caractérisé en ce que**
la partie d'affichage (27) comprend une unité d'imagerie (66) configurée pour imager l'image optique conduite par l'élément (26) conducteur d'image optique,
l'unité d'imagerie (66) est agencée le long de l'axe central de la partie d'insertion (20) de l'endoscope, s'étend dans l'espace interne (64) de la partie d'affichage (27) et fait saillie dans l'espace interne (61) de la partie d'actionnement (21),
la partie d'extrémité de couplage de l'élément (26) conducteur d'image optique est couplée à une partie d'extrémité saillante de l'unité d'imagerie (66),
la partie enroulée (87) est formée d'une partie (87) à petit diamètre prévue au niveau d'une partie périphérique externe de l'unité d'imagerie (66),
l'endoscope comprend une partie (88) de réception de partie d'enroulement prévue dans l'espace interne (61) de la partie d'actionnement (21) ou l'espace interne (64) de la partie d'affichage (27), agencée entre la partie d'enroulement (92) et l'élément (26) conducteur d'image optique et configurée pour limiter le déplacement de la partie d'enroulement (92) vers l'élément (26) conducteur d'image optique, et
la partie (88) de réception de partie d'enroulement est formée d'une partie (88) d'augmentation de diamètre prévue au niveau de la partie périphérique externe de l'unité d'imagerie (66), agencée plus près de la partie d'actionnement (21) que la partie (87) à petit diamètre et présente un diamètre externe plus grand qu'un diamètre externe de la partie (87) à petit diamètre.
